# DEMANDE DE BREVET EUROPEEN

(11) **EP 1 800 714 A1**
(43) Date de publication de la demande: **27.06.2007**
(21) Numéro de dépôt: 06291940.2
(22) Date de dépôt: 15.12.2006
(51) Int. Cl.: A61Q 1/00, A61Q 1/02, A61Q 19/08, A61Q 19/00, A61K 8/25, A61K 8/04, A61K 8/58, A61K 8/81, A61K 8/893, A61K 8/897, A61K 8/896

(54) **Composition cosmétique comprenant des particules concaves et un dispersant, procédés et utilisations**

(30) Priorité: 21.12.2005 FR 0513091
(71) Demandeur: L'Oréal, 75008 Paris (FR)
(72) Inventeur: Cassin, Guillaume, 91140 Villebon sur Yvette (FR); Poret Fristot, Sylvie, 94150 Rungis (FR)
(74) Mandataire: Bulle, Françoise

(57) **Abrégé**

La présente demande concerne une composition cosmétique contenant des particules concaves, au moins un dispersant de ces particules concaves, et éventuellement des particules poreuses. Cette composition est non pulvérulente. La présente demande concerne encore un procédé de traitement cosmétique mettant en oeuvre cette composition ainsi que les utilisations de cette composition notamment pour conférer aux matières kératiniques un aspect mat et/ou pour estomper les imperfections du relief de la peau et/ou cacher les microreliefs, les rides, les ridules et les pores de la peau.

## Description

La présente invention se rapporte à de nouvelles compositions cosmétiques de traitement, de soin ou de maquillage des matières kératiniques humaines telles que la peau, en particulier à des compositions destinées à matifier les matières kératiniques humaines. Les compositions selon la présente demande comprennent des particules concaves et au moins un dispersant de ces particules concaves.

Les compositions matifiantes sont des compositions qui empêchent le support sur lesquelles elles sont appliquées de briller. Appliquée sur le visage, une composition matifiante empêche la peau de briller et unifie le teint. Les compositions de traitement ou de soin de la peau qui présentent des propriétés matifiantes sont généralement utilisées pour résoudre les problèmes de brillance générés par un excès de sébum. Les compositions de maquillage matifiantes visent à améliorer la base du maquillage et donc à améliorer sa tenue. Les compositions matifiantes donnent un aspect mat à la peau dû à la diffusion de la lumière à la surface de la peau. Ces compositions peuvent également être utilisées pour estomper les défauts de la peau comme les microreliefs, les rides, les ridules, les pores ou les variations de couleur.

De façon générale, les compositions cosmétiques matifiantes sont formulées à l'aide de charges minérales (ZnO, SiO₂, ...) enrobées ou non, et d'amidon. Ces particules sont très connues et très employées mais présentent l'inconvénient de sédimenter et sont caractérisées par un toucher rêche peu agréable.

Des compositions matifiantes à base de dispersions aqueuses de styrène/acrylique (FR 2 801 215), de dispersions aqueuses de polytétrafluoroéthylène (FR 2 820 977) et de billes de celluloses de diamètre inférieur à 10µm (FR 2 846 878) sont également connues.

Le problème posé dans la présente demande est l'obtention de compositions cosmétiques présentant des propriétés matifiantes satisfaisantes mêmes lorsqu'elles comprennent une quantité importante de charges matifiantes.

En effet lors de la préparation d'une composition matifiante, il est souvent difficile d'introduire les charges matifiantes sans que celles-ci ne s'agglomèrent. Or l'apparition d'agglomérats a pour conséquence de conduire à une composition non homogène dont les propriétés matifiantes ne sont pas satisfaisantes.

Les compositions matifiantes comprenant une quantité importante de charges présentent en outre généralement un effet sec et poudré trop important.

De manière avantageuse et inattendue, la Demanderesse vient maintenant de découvrir qu'il est possible d'obtenir des compositions cosmétiques présentant des propriétés matifiantes satisfaisantes en combinant des particules concaves et au moins un dispersant de ces particules concaves.

Les compositions selon la présente demande comprennent des particules concaves dispersées de façon homogène. Lors de la préparation de ces compositions on n'observe pas d'agglomérats de particules concaves, il est donc possible d'obtenir des compositions matifiantes comprenant des concentrations élevées de particules concaves.

Par ailleurs, les compositions selon la présente demande présentent des propriétés matifiantes satisfaisantes même lorsque elles contiennent des faibles concentrations de particules concaves. En conséquence les compositions selon la présente demande possèdent également un avantage sur le plan économique.

En outre les caractéristiques organoleptiques de ces compositions, et notamment leur toucher, sont également satisfaisantes.

D'autres caractéristiques, aspects, objets et avantages de la présente invention apparaîtront encore plus clairement à la lecture de la description et des exemples qui suivent.

La présente demande vise une composition cosmétique non pulvérulente contenant des particules concaves et au moins un dispersant de ces particules concaves.

Au sens de la présente demande, un « dispersant des particules concaves » est un composé qui, lorsqu'il est introduit en une teneur de 2% en poids par rapport au poids total de la composition, dans les conditions normales de température et de pression, dans une émulsion huile-dans-eau contenant 1,00% en poids de polyacryldiméthyltauramide d'ammonium, 5,0% en poids de cyclohexasiloxane, 1,70% en poids de glycérine, 0,30% en poids d'alcool stéarylique, 0,70% en poids de Glyceryl stearate / PEG 100 stéarate, 0,50% en poids de dimyristyl tartrate/alcool cetearylique/C12-15 pareth-7/PPG25 laureth-25, 1,5% en poids de particules concaves, et 0,5% en poids de conservateur (le complément à 100% en poids étant de l'eau) par rapport au poids total de la composition, il est solubilisé et permet de diminuer la valeur de R -qui est le rapport entre la réflexion spéculaire et la réflexion diffuse- de cette composition, de préférence, il permet de diminuer la valeur de R à une valeur inférieure ou égale à 1.

La valeur de R définit le caractère matifiant des compositions, elle est définie ci-dessous.

Le caractère matifiant des compositions selon la présente demande est défini par une mesure au gonioréflectomètre. Pour ceci, on étale la composition sur une carte de contraste (Prufkarte type 24/5 - 250 cm² commercialisée par la société Erichsen) à l'aide d'un tire-film mécanique (épaisseur humide 30 microns). On sèche ensuite la composition pendant une nuit à une température de 37 °C, puis on mesure la réflexion à l'aide d'un gonioréflectomètre. Le résultat obtenu est le rapport R entre la réflexion spéculaire et la réflexion diffuse. La valeur de R est d'autant plus faible que l'effet matifiant est important. Les compositions cosmétiques selon l'invention sont celles qui donnent une valeur de R inférieure à 1, de préférence inférieure à 0,75.

Les compositions selon la présente demande laissent apparaître le grain de la peau tout en en masquant les imperfections, plus généralement elles permettent de corriger les dyschromies de la peau.

En particulier la présente demande vise une composition cosmétique, de préférence non pulvérulente, caractérisée par le fait qu'elle contient, dans un milieu physiologiquement acceptable, des particules concaves et au moins un composé choisi parmi les homopolymères de vinylpyrrolidone ainsi que les polyalkylèneglycols de masse moléculaire inférieure à 20 000 g/mole.

La présente demande vise encore un procédé de traitement cosmétique, de soin ou de maquillage des matières kératiniques, notamment de la peau, consistant à appliquer sur lesdites matières kératiniques la composition selon la présente demande, en particulier elle vise un procédé de traitement cosmétique - c'est-à-dire non-thérapeutique - destiné à conférer aux matières kératiniques un aspect mat et/ou à estomper les imperfections du relief de la peau et/ou à cacher les microreliefs, les rides, les ridules et les pores de la peau.

La présente demande vise aussi l'utilisation cosmétique de la composition cosmétique selon la présente demande pour conférer aux matières kératiniques un aspect mat et/ou pour estomper les imperfections du relief de la peau et/ou cacher les microreliefs, les rides, les ridules et les pores de la peau.

La composition selon l'invention comprend des particules concaves. Ces particules ont donc une surface présentant un arrondi intérieur.

Les particules concaves sont en particulier des portions de sphères creuses constituées d'un matériau, notamment d'un matériau organosiliconé.

Lesdites particules concaves ont avantageusement un diamètre moyen allant de 0,05 µm à 20 µm.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau. Il s'agit notamment de demi-sphères creuses.

Le matériau organosiliconé est, de préférence, un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif ou un groupe organique non réactif, de préférence il s'agit d'un groupe organique non réactif.

Le groupe organique non réactif peut être un groupe alkyle en C₁-C₄, notamment un groupe méthyle, éthyle, propyle, butyle, ou un groupe phényle, de préférence c'est un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif est un groupe époxy, un groupe (méth)acryloxy, un groupe alkényle, un groupe mercaptoalkyle, aminoalkyle. Lorsque le groupe organique réactif comprend un radical alkyle, ce radical alkyle comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.

Comme groupe (méth)acryloxy, on peut citer un groupe 3-méthacryloxypropyl, un groupe 3-acryloxypropyl.

Comme groupe alkényle, on peut citer un groupe vinyl, allyl, isopropényl.

Comme groupe mercaptoalkyle, on peut citer un groupe mercaptopropyl, mercaptoéthyl.

Comme groupe aminoalkyle, on peut citer un groupe 3-(2-aminoéthyl)aminopropyl, un groupe 3-aminopropyl, un groupe N,N-diméthylaminopropyl.

Comme groupe halogénoalkyle, on peut citer un groupe 3-chloropropyl, un groupe trifluoropropyl.

Comme groupe glycéroxy, on peut citer un groupe 3-glycéroxypropyl, un groupe 2-glycéroxyéthyl.

Comme groupe uréido, on peut citer un groupe 2-uréidoéthyl.

Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules de matériau organosiliconé peuvent être obtenues selon le procédé décrit ci-dessous comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium, (le groupe R correspondant au groupe R¹ défini précédemment) ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/55, et est préférentiellement de 40/60.

Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30 °C pour éviter une condensation prématurée des composés.

Pour les groupements X et Y des composés (III) et (IV) :
comme groupe alcoxy en C₁-C₄, on peut citer les groupes méthoxy, éthoxy ;
comme groupe alkoxyéthoxy renfermant un groupe alcoxy en C₁-C₄, on peut citer les groupes méthoxyéthoxy, butoxyéthoxy ;
comme groupe acyloxy en C₂-C₄, on peut citer les groupes acétoxy, propionyloxy ;
comme groupe N,N-dilakylamino renfermant un groupement alkyle en C₁-C₄, on peut citer les groupes diméthylamino, diéthylamino ;
comme atome d'halogène, on peut citer les atomes de chlore, de brome ;
comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropionyloxysilane, , le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges. De manière encore préférée, il s'agit du tétraéthoxysilane.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropionyloxysilane, , le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréïdo comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane. Les particules concaves utilisées dans les compositions de la présente demande ont donc avantageusement une structure réticulée constituée d'unités SiO₂ et d'unités RSiO_{1,5} où R est préférentiellement soit un groupe méthyle soit un groupement hydroxyle.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toluène sulfonique, l'acide dodécylbenzènesulfonique, l'acide dodécylsulfonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

Les particules obtenues ont de préférence un diamètre moyen allant de 0,05 à 20 µm.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.

Des portions de sphères creuses en forme de fer à cheval sont aussi décrites dans la demande JP-A-2000-191789.

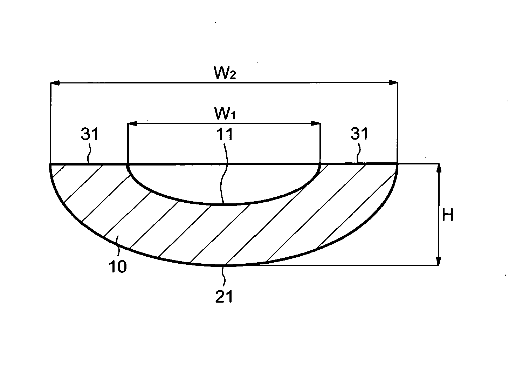

La figure annexée illustre une particule concave de portions de sphères en forme de bol en coupe transversale.

Comme il ressort de cette figure, ces portions concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 20 µm, de préférence de 0,06 à 15 µm en moyenne et la hauteur (H) du grand arc externe (21) allant de 0,015 à 15 µm, de préférence de 0,03 à 15 µm en moyenne.

Les dimensions mentionnées ci-dessus sont obtenues en calculant la moyenne des dimensions de cent particules choisies sur une image obtenue au microscope électronique à balayage.

Comme particules concaves utilisables selon l'invention, on peut citer :
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat), ces particules seront utilisées de manière préférée dans les compositions selon la présente demande ;
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,5 µm et d'épaisseur 350 nm ;
les particules constituées de l'organosilicone réticulé TAK-110 (polymère réticulé méthylsilanol/silicate) de la société TAKEMOTO OIL & FAT , en forme de bol , de largeur 0,7 µm, de hauteur 0,35 µm et d'épaisseur 100 nm ;
les particules constituées de l'organosilicone réticulé TAK-110 (polymère réticulé méthylsilanol/silicate) de la société TAKEMOTO OIL & FAT , en forme de bol , de largeur 7,5 µm, de hauteur 3,5 µm et d'épaisseur 200 nm.

Avantageusement, les particules concaves, notamment les portions de sphères creuses, sont présentes dans la composition selon l'invention, en une teneur allant de 0,01 % à 20 % en poids, de préférence allant de 0,5 % à 10 % en poids, et préférentiellement allant de 0,5 % à 5 % en poids par rapport au poids total de la composition.

En tant que « dispersant de particules concaves » utilisable dans les compositions selon la présente demande, on entend tout composé susceptible de conduire à une répartition homogène des particules concaves dans le milieu de la composition.

Les dispersants de particules concaves utilisables dans les compositions selon la présente demande sont généralement des polymères hydrosolubles ou liposolubles, de manière avantageuse ces dispersants sont les homopolymères de vinylpyrrolidone ainsi que les polyalkylèneglycols de masse moléculaire inférieure à 20 000 g/mole.

Selon un mode particulièrement préféré de l'invention, les homopolymères de vinylpyrrolidone utilisables dans les compositions selon la présente demande ont une masse molaire inférieure à 20 000 g/mole, de préférence inférieure à 10 000 g/mole.

A titre d'homopolymères de vinylpyrrolidone utilisables dans les compositions selon la présente demande, on peut citer les polymères suivants :

| Nom commercial | Masse molaire moyenne en nombre (g/mol) | Fournisseur |
|---|---|---|
| Luviskol K 17 poudre | 2500 | BASF |
| Kollidon 17 PF | 2500 | BASF |
| Kollidon 12 PF | 2500 | BASF |
| Kollidon 12 PF | 10000 | BASF |

Les polyalkylèneglycols utilisables dans les compositions selon la présente demande sont choisis de préférence parmi ceux dont le groupe alkylène comprend de 1 à 4 atomes de carbone notamment les polyéthylèneglycols, les polypropylèneglycols et les polybutylèneglycols. Ces polyalkylèneglycols présentent une masse moléculaire inférieure à 20 000 g/mole, de préférence inférieure à 10 000 g/mole. A titre d'exemple, on peut citer le polyéthylèneglycol de masse moléculaire 1450 g/mole fourni sous la dénomination « Sentry 10 Polyethylène Glycol » par la société UNION CARBIDE.

Avantageusement les dispersants de particules concaves, en particulier les homopolymères de vinylpyrrolidone et les polyalkylèneglycols de masse moléculaire inférieure à 20 000 g/mole sont utilisés en une quantité allant de 0,01 % à 10 %, de préférence allant de 0,1 % à 5 %, et de manière encore plus préférée de 0,2 % à 2,5 % en poids par rapport au poids total de la composition.

Selon un mode de réalisation particulier avantageux, les compositions selon la présente demande comprennent des charges poreuses. L'introduction de particules poreuses dans une composition selon la présente demande comprenant des particules concaves et au moins un dispersant de ces particules concaves permet d'augmenter le caractère matifiant de ladite composition ; ou pour une valeur de matité donnée, l'introduction de particules poreuses dans une composition selon la présente demande permet d'utiliser une quantité moindre de particules concaves.

Au sens de la présente demande, le terme « charge poreuse » désigne toute particule sphérique ou non, poreuse, non soluble dans les compositions selon la présente demande et différente des particules concaves utilisées dans les compositions selon la présente demande.

Lesdites charges poreuses utilisables dans les compositions de la présente demande sont des particules solides, non solubles dans les conditions normales de température et de pression dans le milieu où elles sont introduites, qui, lorsqu'elles sont ajoutées dans une émulsion huile-dans-eau contenant 1,00 % en poids de polyacryldiméthyltauramide d'ammonium, 5,0 % en poids de cyclohexasiloxane, 1,70 % en poids de glycérine, 0,30 % en poids d'alcool stéarylique, 0,70 % en poids de glycéryl stéarate / PEG 100 stéarate, 0,50 % en poids de dimyristyl tartrate/alcool cetearylique/C12-15 pareth-7/PPG25 laureth-25, 1,5 % en poids de particules concaves, 2 % en poids de polyvinylpyrrolidone et 0,50 % en poids de conservateurs (le complément à 100 % en poids étant de l'eau) par rapport au poids total de la composition, permettent de diminuer la valeur de R de cette composition, de préférence elles permettent de diminuer la valeur de R à une valeur inférieure ou égale à 1, de manière encore plus préférée à une valeur inférieure ou égale à 0,75.

Les charges poreuses présentent avantageusement une taille volumique inférieure à 40 µm en particulier inférieure à 30 µm, voire inférieure à 15 µm. D'une manière générale, la composition selon l'invention peut contenir diverses charges d'origine minérale ou organique. Ces charges peuvent être de toute forme, notamment elles peuvent être plaquettaires, sphériques, oblongues... Ces charges peuvent être de toute forme cristallographique notamment en feuillets, cubique, hexagonale, orthorombique... Ces charges sont poreuses, cette porosité peut notamment se traduire par une surface spécifique des particules supérieure à 10m²/g, et en particulier supérieure à 50m²/g.

De préférence les charges poreuses sont sphériques.

Selon un autre mode préféré, ces charges poreuses sont de nature inorganique.

Plus particulièrement, ces charges peuvent par exemple être choisie parmi :
- les microparticules poreuses de silice, comme par exemple les Silica beads SB150 et SB700 de Miyoshi de taille moyenne 5 microns; les Sunspheres Série-H d'Asahi Glass comme par exemple les Sunsphères H33, H51, et H53 de tailles respectives 3, 5 et 5 microns ;
- les poudres de copolymères acryliques, notamment de poly(méth)acrylate de méthyle (PMMA), comme par exemple les sphères creuses de PMMA vendues sous la dénomination Covabead LH85 par la société Wackherr ;
- les poudres composites de talc/dioxyde de titane/alumine/silice comme celles vendues sous la dénomination Coverleaf AR-80 par la société Catalyst & Chemicals ;
- les poudres de polyamide (par exemple Nylon®), comme par exemple les particules de Nylon 12 du type Orgasol d'Atofina de taille moyenne 10 microns;
- et leurs mélanges.

Les taux introduits dépendent de l'effet recherché.

Avantageusement les charges poreuses sont utilisées en une quantité allant de 0,1 % à 10 %, de préférence allant de 0,5 % à 5 %, et de manière encore plus préférée de 0,5 % à 3 en poids par rapport au poids total de la composition.

La composition conforme à l'invention peut également contenir au moins un actif cosmétique.

Parmi les principes actifs cosmétiques utilisables dans les compositions selon la présente demande, on compte les antioxydants, les filtres solaires physiques et chimiques, les vitamines (notamment C, B3, PP, B5, E, K1 et leurs dérivés comme leurs esters), les actifs hydratants comme les polyols et notamment la glycérine, les composés auto-bronzants, les actifs antirides, les céramides, la DHEA et ses dérivés, le coenzyme Q10, les agents anti-pollution ou anti-radicaux libres, les actifs dermo-relaxants, les agents apaisants, les agents stimulant la synthèse de macromolécules dermiques ou épidermiques et/ou empêchant leur dégradation, les agents anti-glycation, les agents anti-irritants, les agents desquamants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

De manière particulière les compositions selon la présente demande contiennent un actif cosmétique anti-âge choisi parmi les composés augmentant la synthèse de collagène, les composés inhibant la dégradation du collagène, les agents dermo-décontractants, les agents inhibant la glycation des protéines, les agents augmentant la prolifération des kératinocytes, les agents augmentant la différenciation des kératinocytes, et leurs mélanges.

De manière particulière les compositions selon la présente demande contiennent un principe actif cosmétique blanchissant.

Parmi les principes actifs cosmétiques blanchissant utilisables dans les compositions selon la présente demande, on compte les agents dépigmentant, anti-pigmentant ou pro-pigmentant et leurs mélanges.

Les agents dépigmentants ou anti-pigmentants susceptibles d'être incorporés dans la composition selon la présente demande comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

Parmi les principes actifs cosmétiques anti-âge utilisables dans les compositions selon la présente demande, on compte les composés suivants :
- les composés augmentant la synthèse de collagène (tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes) et/ou d'élastine (tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie®) et/ou des glycosaminoglycannes (tels que le produit de fermentation du lait par Lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin®) et/ou des protéoglycannes et/ou de la fibronectine (tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®) et/ou de la laminine ;
- les composés inhibant la dégradation du collagène (tels que les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja commercialisés par exemple par la société ICHIMARU PHARCOS sous la dénomination commerciale Flavostérone SB®, de trèfle rouge, de lin, de kakkon ou de sauge) et/ou de l'élastine (tels que l'extrait peptidique de graines de Pisum sativum commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique) ;
- les agents dermo-décontractants, tels que l'alvérine et ses sels, les sels de manganèse et en particulier le gluconate de manganèse, les sels de magnésium et en particulier le gluconate et le sulfate de magnésium, l'hexapeptide Argireline R commercialisé par la société LIPOTEC, l'adénosine, ainsi que les sapogénines et les extraits naturels, en particulier de Dioscorea opposita ou de Dioscorea villosa (Wild Yam) en contenant, ainsi que les extraits de Boswellia serrata ;
- les agents inhibant la glycation des protéines, tels que les extraits végétaux de la famille des Ericaceae, notamment un extrait de myrtille (Vaccinium angusfifollium) ; l'ergothionéine et ses dérivés ; et les hydroxystilbènes et leurs dérivés, tels que le resvératrol et le 3,3', 5,5'-tétrahydroxystilbène ;
- les agents augmentant la prolifération des kératinocytes (tels que les rétinoïdes dont le rétinol et le palmitate de rétinyle, l'adénosine, le phloroglucinol, les extraits de tourteaux de noix commercialisés par la société GATTEFOSSE et les extraits de Solanum tuberosum commercialisés par la société SEDERMA) et/ou des fibroblastes (tels que les protéines ou polypeptides végétaux, extraits notamment du soja, et les hormones végétales telles que les giberrellines et les cytokinines) ;
- les agents augmentant la différenciation des kératinocytes, tels que les minéraux dont le calcium ; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol ; et leurs mélanges.

De manière particulière les compositions selon la présente demande contiennent un principe actif pour le traitement cosmétique des peaux grasses.

Parmi les actifs pour le traitement cosmétique des peaux grasses, on compte les sels de zinc et en particulier le gluconate de zinc ; les antibactériens comme l'acide salicylique, le triclosan, le lipacide, l'extrait de clou de girofle, l'octopirox, l'hexamidine, les actifs anti acné.

La composition selon l'invention comprend de 0,001 à 10 %, de préférence de 0,01 à 5 % en poids d'au moins un principe actif par rapport au poids total de la composition.

La composition conforme à l'invention peut également contenir au moins un additif cosmétique choisi parmi les colorants, les pigments, les parfums, les agents tenseurs, les conservateurs, les séquestrants, les actifs liposolubles ou hydrosolubles, les ajusteurs de pH (acides ou bases), les agents gélifiants et/ou épaississants hydrophiles ou lipophiles, les émollients, les agents filmogènes, les tensioactifs et leurs mélanges.

Les additifs cosmétiques sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Les compositions cosmétiques décrites dans la présente invention peuvent se présenter sous la forme d'émulsions directes inverses ou multiples (eau dans huile dans eau ou bien huile dans eau dans huile), de gels-crèmes, de lotions, de sticks, des sprays, de compositions anhydres.

Les exemples qui suivent servent à illustrer l'invention sans toutefois présenter un caractère limitatif.

### EXEMPLES

### I. Préparation des compositions

Les compositions suivantes ont été préparées.
Les quantités ci-dessous sont exprimées en pourcentages en poids par rapport au poids total de la composition.

| **exemple** | **1** (comparatif) | **2** | **3** | **4** (comparatif) |
|---|---|---|---|---|
| Polyacryldiméthyl tauramide d'ammonium (1) | 1,00% | 1,00% | 1,00% | 1,00% |
| cyclohexasiloxane | 5,0% | 5,0% | 5,0% | 5,0% |
| Glycérine | 1,70% | 1,70% | 1,70% | 1,70% |
| Alcool stéarylique | 0,30% | 0,30% | 0,30% | 0,30% |
| Glyceryl stearate / PEG 100 stéarate | 0,70% | 0,70% | 0,70% | 0,70% |
| dimyristyl tartrate/alcool cetearylique/C 12-15 pareth-7/PPG25 laureth-25 | 0,50% | 0,50% | 0,50% | 0,50% |
| Particules concaves d'organopolysiloxane (2) | 1,50% | 1,50% | 1,50% | - |
| polyvinylpyrrolidone | - | 2% | 2% | 2% |
| Particules poreuses (3) | - | - | 1,50% | 1,50% |
| Conservateur | 0,50% | 0,50% | 0,50% | 0,50% |
| eau | Qsp 100 | Qsp 100 | Qsp 100 | Qsp 100 |

(1) : Hostacerin AMPS de Clariant
(2) : NLK-506 par la société Takemoto Oil & Fat
(3) : Particules poreuses de silice SB700 de Miyoshi

On prépare les compositions des exemples suivant en ajoutant, sous agitation, la phase huileuse chauffée à environ 65°C à la phase aqueuse chauffée à la même température.

### II. Test de matité

La matité des compositions des exemples 1 à 4 a été mesurée conformément au protocole exposé ci-avant qui met en oeuvre une mesure au gonioréflectomètre sur une carte de contraste (Prufkarte type 24/5 - 250 cm² commercialisée par la société Erichsen). Le résultat obtenu est le rapport R entre la réflexion spéculaire et la réflexion diffuse. La valeur de R est d'autant plus faible que l'effet matifiant est important.

| **Composition** | **Exemple 1** (comparatif) | **Exemple 2** | **Exemple 3** | **Exemple 4** (comparatif) |
|---|---|---|---|---|
| R | 1,30±0,10 | 0,92±0,03 | 0,67±0,01 | 1,85±0,05 |

Ces résultats in vitro montrent qu'en combinant les particules concaves d'organopolysiloxane avec un homopolymère de vinylpyrrolidone (exemples 2 et 3), on obtient un résultat de matité supérieur à celui obtenu avec les compositions comparatives. De plus l'utilisation d'une composition de particules concaves d'organopolysiloxane avec un homopolymère de vinylpyrrolidone et des charges poreuses (exemple 3) permet l'obtention de propriétés matifiantes supérieures. En comparaison, l'association de charges poreuses de silice avec un homopolymère de vinylpyrrolidone ne permet pas d'accéder à des résultats satisfaisants.

### III .Evaluation sensorielle :

L'effet matifiant et les aspects cosmétiques de la composition de l'exemple 3 ont été évalués sur un panel de 7 femmes ayant une peau grasse. Pour ces femmes présentant une peau brillante, la composition de l'exemple 3 a pour effet de matifier et d'affiner le grain de la peau, les pores semblent moins dilatés et le teint plus unifié.

## Revendications

1. Composition cosmétique non pulvérulente **caractérisée par le fait qu'**elle contient des particules concaves et au moins un dispersant de ces particules concaves.

2. Composition cosmétique **caractérisée par le fait qu'**elle contient, dans un milieu physiologiquement acceptable, des particules concaves et au moins un composé choisi parmi les homopolymères de vinylpyrrolidone ainsi que les polyalkylèneglycols de masse moléculaire inférieure à 20 000 g/mole.

3. Composition selon l'une quelconque des revendications précédentes, **caractérisée par le fait que** lesdites particules concaves ont un diamètre moyen allant de 0,05 µm à 20 µm.

4. Composition selon l'une quelconque des revendications précédentes **caractérisée par le fait que** les particules concaves sont sous forme de portions de sphères creuses présentant une section transversale en forme de fer à cheval ou d'arceau.

5. Composition selon l'une quelconque des revendications précédentes, **caractérisée par le fait que** les particules concaves sont constituées d'un matériau organosiliconé.

6. Composition selon la revendication précédente, **caractérisée par le fait que** le matériau organosiliconé est un polysiloxane réticulé de structure tridimensionnelle comprenant, ou constitué de, des motifs de formule (I) : SiO₂ et de formule (II) : R¹SiO_{1,5}
dans lesquelles R¹ désigne un groupe organique ayant un atome de carbone directement relié à l'atome de silicium.

7. Composition selon la revendication précédente, **caractérisée par le fait que** le groupe organique est un groupe organique réactif, un groupe organique non réactif, et de préférence un groupe organique non réactif.

8. Composition selon la revendication précédente, **caractérisée par le fait que** le groupe organique non réactif peut être un groupe alkyle en C₁-C₄, ou un groupe phényle.

9. Composition selon la revendication 7 ou 8, **caractérisée par le fait que** le groupe organique non réactif est un groupe méthyle.

10. Composition selon la revendication 7, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

11. Composition selon la revendication 7 ou 10, **caractérisée par le fait que** le groupe organique réactif est choisi parmi un groupe époxy, un groupe (méth)acryloxy, un groupe alkényle, un groupe mercaptoalkyle, aminoalkyle.

12. Composition selon l'une quelconque des revendications 6 à 11, **caractérisée par le fait que** le matériau organosiliconé comprend les motifs (I) et (II) selon un rapport molaire motif (I) / motif (II) allant de 30/70 à 50/50, de préférence allant de 35/65 à 45/55.

13. Composition selon l'une quelconque des revendications 6 à 12, **caractérisée par** le fait les particules d'organosilicone sont susceptibles d'être obtenues selon un procédé comprenant :
a. l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
b. la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

14. Composition selon la revendication précédente, **caractérisée par le fait que** dans l'étape (a), le rapport molaire du composé (III) au composé (IV) va de 30/70 à 50/50, de préférence va de 35/65 à 45/55, et préférentiellement est de 40/60.

15. Composition selon la revendication 13 ou 14, **caractérisée par le fait que** le rapport en poids de l'eau au total des composés (III) et (IV) va de 10/90 à 70/30 dans l'étape (a).

16. Composition selon la revendication 14, **caractérisée par le fait que** le groupe organique est un groupe organique réactif, un groupe organique non réactif, et de préférence un groupe organique réactif.

17. Composition selon la revendication 13, **caractérisée par le fait que** le groupe organique non réactif peut être un groupe alkyle en C₁-C₄, ou un groupe phényle.

18. Composition selon la revendication 13 ou 17, **caractérisée par le fait que** le groupe organique non réactif est un groupe méthyle.

19. Composition selon la revendication 16, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

20. Composition selon la revendication 16 ou 19, **caractérisée par le fait que** le groupe organique réactif est choisi parmi un groupe époxy, un groupe (méth)acryloxy, un groupe alkényle, un groupe mercaptoalkyle, aminoalkyle.

21. Composition selon l'une quelconque des revendications 13 à 20, **caractérisée par le fait que** les catalyseurs d'hydrolyse et de polymérisation sont indépendamment choisis parmi l'hydroxyde de sodium, l'hydroxyde de potassium, le carbonate de sodium, l'hydrogénocarbonate de sodium, l'ammoniaque, la triméthylamine, la triéthylamine, l'hydroxyde de tétraméthylammonium, l'acide citrique, l'acide acétique, l'acide méthanesulfonique, l'acide p-toluène sulfonique, l'acide dodécylbenzènesulfonique, l'acide dodécylsulfonique, l'acide chlorhydrique, l'acide sulfurique, l'acide phosphorique.

22. Composition selon l'une quelconque des revendications précédentes, **caractérisée par le fait que** les particules concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 20 µm, de préférence de 0,06 à 15 µm en moyenne et la hauteur (H) du grand arc externe (21) allant de 0,015 à 15 µm, de préférence de 0,03 à 15 µm en moyenne.

23. Composition selon l'une quelconque des revendications précédentes, **caractérisée par le fait que** les portions de sphères creuses sont présentes en une teneur allant de 0,01 % à 20 % en poids, de préférence allant de 0,5 % à 10 % en poids, et préférentiellement allant de 0,5 % à 5 % en poids par rapport au poids total de la composition.

24. Composition selon la revendication précédente, **caractérisée en ce que** le dispersant de particules concaves est un homopolymère de vinylpyrrolidone de masse moléculaire inférieure à 20 000 g/mole, de préférence inférieure à 10 000 g/mole.

25. Composition selon l'une quelconque des revendications 1 à 23 **caractérisée en ce que** le dispersant de particules concaves est choisi parmi les polyalkylèneglycols dont le groupe alkylène comprend de 1 à 4 atomes de carbone notamment les polyéthylèneglycols, les polypropylèneglycols et les polybutylèneglycols.

26. Composition selon l'une quelconque des revendications précédentes, **caractérisée en ce que** la quantité de dispersants de particules concaves va de 0,01 % à 10 %, de préférence allant de 0,1 % à 5 %, et de manière encore plus préférée de 0,2 % à 2,5 % en poids par rapport au poids total de la composition.

27. Composition selon l'une quelconque des revendications précédentes, **caractérisée en ce qu'**elle comprend des charges poreuses, lesdites charges poreuses étant des particules solides, non solubles dans les conditions normales de température et de pression, qui, lorsqu'elles sont ajoutées à une émulsion huile-dans-eau contenant 1,00 % en poids de polyacryldiméthyltauramide d'ammonium, 5,0 % en poids de cyclohexasiloxane, 1,70 % en poids de glycérine, 0,30 % en poids d'alcool stéarylique, 0,70 % en poids de glycéryl stéarate / PEG 100 stéarate, 0,50 % en poids de dimyristyl tartrate/alcool cetearylique/C12-15 pareth-7/PPG25 laureth-25, 1,5 % en poids de particules concaves, 2 % en poids de polyvinylpyrrolidone et 0,50 % en poids de conservateur par rapport au poids total de la composition, permettent de diminuer la valeur de R de cette composition, ladite valeur R étant le rapport entre la réflexion spéculaire et la réflexion diffuse.

28. Composition selon la revendication 27, **caractérisée en ce que** les charges poreuses sont choisies parmi les microparticules poreuses de silice, les poudres de copolymères acryliques, les poudres composites de talc/dioxyde de titane/alumine/silice, les poudres de polyamide et leurs mélanges.

29. Composition selon l'une quelconque des revendications 27 ou 28, **caractérisée en ce que** la quantité de particules poreuses va de 0,1 % à 10 %, de préférence allant de 0,5 % à 5 %, et de manière encore plus préférée de 0,5 % à 3 % en poids par rapport au poids total de la composition.

30. Composition selon l'une quelconque des revendications précédentes **caractérisée en ce qu'**elle comprend un actif cosmétique.

31. Composition selon la revendication précédente **caractérisée en ce que** l'actif cosmétique est un actif cosmétique anti-âge choisi parmi les composés augmentant la synthèse de collagène, les composés inhibant la dégradation du collagène, les agents dermo-décontractants, les agents inhibant la glycation des protéines, les agents augmentant la prolifération des kératinocytes, les agents augmentant la différenciation des kératinocytes, et leurs mélanges

32. Composition selon la revendication 30 **caractérisée en ce que** l'actif cosmétique est un actif cosmétique blanchissant choisi parmi les agents dépigmentant, anti-pigmentant ou pro-pigmentant et leurs mélanges.

33. Composition selon la revendication 30 **caractérisée en ce que** l'actif cosmétique est choisi parmi les actifs pour le traitement cosmétique des peaux grasses, les antibactériens, les actifs anti-acné.

34. Composition selon la revendication 30 **caractérisée en ce que** l'actif cosmétique est choisi parmi les antioxydants, les filtres solaires physiques et chimiques, les vitamines, les actifs hydratants, les composés auto-bronzants, les actifs antirides, les céramides, la DHEA et ses dérivés, le coenzyme Q10, les agents anti-pollution ou anti-radicaux libres, les actifs dermo-relaxants, les agents apaisants, les agents stimulant la synthèse de macromolécules dermiques ou épidermiques et/ou empêchant leur dégradation, les agents anti-glycation, les agents anti-irritants, les agents desquamants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges

35. Composition selon l'une des revendications 30 à 36 **caractérisée en ce qu'**elle comprend de 0,001 à 10 %, de préférence de 0,01 à 5 % en poids d'au moins un principe actif par rapport au poids total de la composition.

36. Composition selon l'une quelconque des revendications précédentes **caractérisée en ce qu'**elle comprend un additif cosmétique choisi parmi les colorants, les pigments, les parfums, les agents tenseurs, les conservateurs, les séquestrants, les actifs liposolubles ou hydrosolubles, les ajusteurs de pH (acides ou bases), les agents gélifiants et/ou épaississants hydrophiles ou lipophiles, les émollients, les agents filmogènes, les tensioactifs et leurs mélanges.

37. Composition selon l'une quelconque des revendications précédentes **caractérisée en ce qu'**elle se présente sous la forme d'émulsions directes inverses ou multiples, de gels-crèmes, de lotions, de sticks, des sprays, de compositions anhydres.

38. Procédé de traitement cosmétique, de soin ou de maquillage des matières kératiniques, notamment de la peau, consistant à appliquer sur lesdites matières kératiniques une composition selon l'une quelconque des revendications précédentes.

39. Utilisation cosmétique d'une composition selon l'une quelconque des revendications 1 à 37 pour conférer aux matières kératiniques un aspect mat.

40. Utilisation cosmétique d'une composition selon l'une quelconque des revendications 1 à 37 pour estomper les imperfections du relief de la peau et/ou cacher les microreliefs, les rides, les ridules et les pores de la peau.
